Europäisches Patentamt

(19) European Patent Office

Office européen des brevets

(11) Publication number : **0 433 250 A2**

# (12) EUROPEAN PATENT APPLICATION

(21) Application number : 90830588.1

(22) Date of filing : 14.12.90

(51) Int. Cl.⁵ : **A61M 5/50**

(30) Priority : 15.12.89 IT 376989
21.03.90 IT 340090

(43) Date of publication of application :
19.06.91 Bulletin 91/25

(84) Designated Contracting States :
AT BE CH DE DK ES FR GB GR LI LU NL SE

(71) Applicant : IPPOCRATE S.R.L.
Via De Bonomini 69
I-41100 Modena (IT)

(72) Inventor : Barbieri, Giuseppe
Via Pieve Del Pino 38
I-40037 Sasso Marconi, Bologna (IT)
Inventor : Bordini, Deanna
Via Grazia Deledda 2
I-41051 Montale di Castelnuovo Rangone (IT)
Inventor : Gatti, Luciano
Via Grazia Deledda 4
I-41051 Montale di Castelnuovo Rangone (IT)

(74) Representative : Lanzoni, Luciano
c/o BUGNION S.p.A. Via dei Mille, 19
I-40121 Bologna (IT)

(54) **Disposable syringe.**

(57)  This disposable syringe has, inside its cylinder (2) and close to its tapered end (3) with a fitting for the attachment of the base (4a) of an injection needle (4), at least one projection or relief (7) which prevents the plunger (5) from moving away from the needle (4) once the plunger (5) has nearly reached the end of the cylinder (2) and passed the projection (7) ; this syringe also has a guard (8) one end of which flexibly pivots about the said base (4a) and which has at least one, one-way locking element (9) for holding the needle (4) and which only permits the entry of the needle (4) into the guard (8) when the latter automatically moves into its stable position covering the needle (4).

FIG2

EP 0 433 250 A2

# DISPOSABLE SYRINGE

The present invention consists of a disposable syringe which cannot be used more than once and whose re-use is prevented by automatic blocking of the plunger and automatic locking of the needle after a single use. Disposable syringes, also mistakenly known as "single use" syringes, have been known for some time and consist of syringes complete with injection needles which are designed to be thrown away after they have been used once.

This type of syringe consists of a cylinder which has a fitting on one end for the attachment of an injection needle and a sealed plunger which is moved inside the cylinder by a rod which protrudes from the other end of the cylinder. With the exception of the needle, all parts of the syringe are in plastic so that it can be manufactured economically. The syringe complete with needle is packed inside a sealed packet to ensure that it is kept sterile.

When the syringe is required, the packet is torn open, the syringe is ready for aspirating the liquid to be injected and can then be used.

Syringes of this type are extremely practical because they are ready for immediate use and the needle is perfectly sharp because it is only used once. Despite the intentions of the manufacturers, however, these syringes are often re-used.

A further disadvantage of all syringes is their limited degree of safety. Before use, each needle is protected by a cover or a cap which is removed and, in most cases, then thrown away just before the syringe is used. When the injection has been completed the syringe can no longer be used but the needle is without any form of protection whatsoever. If the syringe is not immediately put in a safe place or if its cap is not replaced it can cause injury to the person using it. The safety of syringes is extremely important since injuries caused by needles can lead to the spread of infections and contagious diseases among medical and nursing staff.

The aim of the present invention is to eliminate the above-mentioned disadvantages by providing a disposable syringe which can only be used once even though the user might attempt otherwise and by providing a syringe which gives a certain degree of active, automatic safety, i.e. a syringe which does not require any action on part of the user to protect the needle and render it harmless.

The present invention, described in the claims below, solves the problems mentioned with a disposable syringe which has, inside its cylinder and close to its tapered end with a fitting for the attachment of the base of an injection needle, at least one projection or relief which prevents the plunger from moving away from the needle once the plunger has practically reached the end of the cylinder and passed the projection; this syringe also has a guard one end of which flexibly pivots about the said base and which has at least one, one-way locking element for holding the needle in place ; this locking element only permits the entry of the needle into the guard when the latter automatically moves into its stable position covering the needle.

The characteristics and advantages of the disposable syringe in this invention are explained in greater detail in the following description and in the diagrams enclosed, these being only one example of the invention and not to be interpreted as being in any way restrictive.

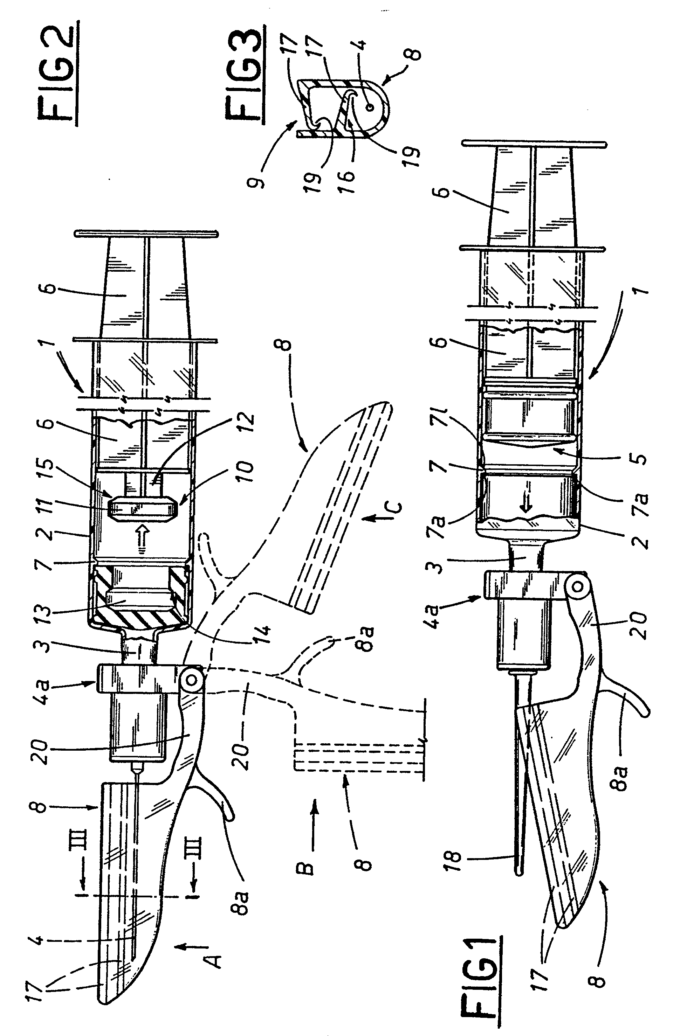

Figures 1 and 2 are side views of the disposable syringe before and after use where some parts have been cut away to show others more clearly ; Figure 3 shows a cross section of the section III-III in figure 2.

With reference to the enclosed figures, the disposable syringe described in this invention is indicated in its entirety by the number 1 and consists of a cylinder 2, containing the liquid to be injected, with a tapered end 3 which has a sealed fitting for the attachment of the base 4a of an injection needle 4 and a sealed piston or plunger 5 which moves inside the cylinder 2 to aspirate and then expel the liquid to be injected. The plunger 5 is made of a soft material, such as rubber or other material used for seals, to ensure a good seal and is fixed to one end of a rod 6 which in turn projects out of the other, free end of the cylinder 2 thus enabling the axial movement of the rod 6 and plunger 5 fixed to it. At the end of the rod 6 which is fixed to the plunger 5 there are means 10 for fixing the plunger 5 to the rod 6. These fixing means 10 usually consist of a mushroom head whose rod 12 is coaxial to the rod 6 and whose free end is fixed to the latter. The mushroom head 11 is housed in a seat 13 made in the plunger 5, the said seat 13 having an undercut 14 designed to intercept the mushroom head 11 when the rod 6 is withdrawn from the cylinder 2.

According to the present invention, the cylinder 2 has, close to its tapered end 3 at least one internal projection 7 designed to permit the free forward movement of the plunger towards the tapered end 3 but also designed to prevent movement of the plunger 5 in the opposite, rearwards direction.

The projection 7 is located at a distance from the end of tapered end 3 which is not less than the length of the plunger 5 measured along the axis of the cylinder 2. In this way the projection 7 acts as a fixed seat to receive the plunger 5.

The fixing means 10 are shaped in such a way that their connection with the plunger 5 is interrupted only when the latter has travelled beyond the projection 7 during its forward movement towards the needle 4. This connection must however remain active when the plunger 5 is moved away from the needle 4 in order to aspirate the injection liquid and is not intercepted by the projection 7.

The projection 7 can take various forms. It could, for example, be a single projection only and cover a small area of the cylinder 2 or it could be a single, continuous ring shaped projection or it could also be a series of projections 7 positioned around the internal circumference of the cylinder 2.

In its preferred form, the projection 7 is a single ring with an approximately triangular cross-section ; one of the sides of the triangle coincides with the generatrix of the cylinder 2 while the other two sides, indicated by 71 and 7a, are at different angles with respect to the said generatrix of the cylinder 2. The section defined by the triangle side 7a is more steeply angled or even perpendicular, for example, with respect to the generatrix of the cylinder 2 and is on the side closest to the needle 4 : in this way when the plunger 5 is moved towards the needle 4 and passes the projection 7, the less steeply angled section 71 does not hinder movement of the plunger 5 but rather causes a gradual reduction in diameter. The section 7a, on the other hand, is an undercut which intercepts the plunger 5 and its angle with respect to the generatrix of the cylinder 2 is such that the force which must be applied to the plunger 5 in order to move it past the projection 7 is greater than the force required to extract the mushroom head 11 from its seat 13 made in the plunger 5. In order to assist the interruption of the connection between the fixing means 10 and the plunger 5, the mushroom head 11 has, on the rod side 6, a truncated-cone shaped or bevelled 15 surface which tapers towards the rod 11. The angle of the surface 15 is such that the pulling force applied to the plunger 5 during aspiration or withdrawal of the injection liquid is less than the force which is required, as the rod 6 is moved away from the needle, to enable the surface 15 to overcome the elastic force exercised on the surface by the undercut 14 of the plunger 5.

In the manufacturing stage, the projection 7 could advantageously be formed by reducing the diameter of the cylinder 2 through permanent plastic deformation : next, the rod 6 and the plunger 5 would be placed inside the cylinder 2 without allowing the plunger 5 to travel beyond the projection 7. This manufacturing method would not require excessive variations to current production techniques for disposable syringes because the plunger 5 in current syringes is not fitted in contact with the tapered end 3 of the cylinder 2 in order to prevent adhesion between the end and the plunger.

During use the plunger, 5 is drawn away from the needle 4 so as to create a vacuum inside the cylinder 2 sufficient to aspirate the injection liquid : the projection 7 reduces the volume of liquid which can be aspirated into the cylinder 2 but this is not a problem because the capacity of syringes currently being used is always greater than the volume of liquid these syringes are designed to aspirate. To inject the liquid contained in the cylinder 2, the rod 6 is pushed so that the plunger 5 moves towards the needle 4. At a certain point in its travel, the plunger intercepts the least steeply angled section 71 of the projection 7. When all the liquid has been injected, the plunger 5 will have completely passed the projection 7. If at this point an attempt is made to re-use the syringe by pulling the rod 6 and consequently the plunger 5 backwards beyond the projection 7, the more steeply angled section 7a of the projection 7 will stop further backwards movement of the plunger and cause the mushroom head 11 to leave its seat 13 in the plunger 5.

One end of a guard 8 pivots on the base 4a of the needle 4. The guard 8 has a "U" shaped cross-section at least on that section which, as explained below, engages with the needle 4. The guard is permanently sprung with, for example, a needle spring hidden in the pivot point, and moves between a position which is made unstable by the spring and where it is at an angle to the cylinder 2 and a stable position where it covers the needle 4 (see fig. 2 where this position is indicated by A).

The length of the guard 8 is such that in its stable position A it at least covers the entire length of the needle 4.

The inside of the guard 8 has at least one, one-way locking element 9 running the length of the guard 8 which is flexible enough to permit the entry of the needle 4 into the guard 8 only when the latter springs into its stable position. Obviously because the locking element 9 is one-way it prevents the needle 4 from being withdrawn from the guard 8 once the needle 4 has passed the locking element 9 as it travels towards the inside of the guard 8.

In figure 3 there are two, one-way locking elements 9 and 16 both consisting of a strip 17 which extends lengthways inside the guard 8 at an angle with respect to the guard arms. Each strip 17 is fixed to its own arm on the guard 8 along its outermost lengthways edge ; the free lengthways edge of the each strip skims or is in complete contact with the other arm of the guard 8. As can be seen in figure 3, the two strips 17 are independent and at an angle to each other and act independently on the needle 4.

The flexibility of the strips 17 is such that they permit the passage, in one direction only, of the needle 4 itself but not of the needle 4 fitted with a protective cap or covering 18 prior to use.

In order to ensure greater efficiency, the free lengthways edge of each strip 17 is bent towards the inside of the guard 8 so as to form a hook 19 which prevents withdrawal of the needle 4 from the guard 8 once it has passed the strips 17.

In order to facilitate use of the syringe 1 described in this invention, the side of guard 8 facing the cylinder 2 when the guard is in its stable position, has a handle recess 20 for holding the guard in its unstable position, indicated by B in figure 2. The guard 8 can also be pivoted backwards through 180$^{1}$ with respect to the cylinder 2 to its unstable position so that it nearly makes contact with the cylinder ; this position is indicated by C in figure 2. The disposable syringe 1 manufactured according to the present invention is ready for use with the needle 4 protected and contained in a cap 18 which prevents the needle 4 from entering the sprung guard 8 which presses against the needle. The syringe 1 is prepared for use by folding the guard 8 backwards and removing the cap 18. Once the syringe has been used it can safely be put down because the springs immediately returns the guard 8 to its stable, rest, non-use position shown by A in figure 2. During this movement of the guard 8, the flexible strips 17 pass over and trap the needle 4 : any attempts to now fold the guard back towards its unstable position in order to free the needle and re-use the syringe 1 are prevented by the resistance of the strips 17 ; such attempts will only succeed in bending the needle 4.

It is clear therefore that the disposable syringe described in the present invention is truly a safe, single-use syringe firstly, because the plunger 5 cannot be moved backwards to enable re-use and secondly, because the guard 8 fully and irreversibly covers the needle 4 after use.

One of the main advantages of the syringe in the present invention is that it is extremely simple to manufacture and use. This simplicity enables considerable production economies which are also to the advantage of users.

This invention can be subject to numerous modifications and variations, all of which enter within the terms of the invention. For example, the guard 8, could be fitted with a grip 8a to facilitate positioning of the guard during use of the syringe. All the details of this invention can be substituted by their technical equivalents.

In practice, modifications and improvements are of course possible and enter within the terms of the following claims.

**Claims**

1. A disposable, single-use syringe consisting of :
   – a cylinder (2) for containing the liquid to be injected, having at one end a tapered end (3) with a sealed fitting for attachment to the base (4a) of an injection needle (4) ; and
   – a piston or plunger (5) sealed and moving inside a cylinder (2) and fixed to the end of a rod (6) which projects from the other, free end of the cylinder (2) ; the plunger (5) is made in a soft material and held by fixing means (10) on the end of the rod (6) ; before the syringe (1) is used, the said plunger (5) is at a particular distance from the tapered end (3) of the cylinder (2), wherein the said cylinder (2) has, close to its end with the tapered end (3) at least one internal projection or relief (7) designed to intercept the outside of the said plunger (5) as it moves towards and away from the said needle (4) ; the said projection (7) is shaped in such a way that it prevents movement of the plunger (5) only when the latter is being moved away from the needle (4) and once the plunger (5) has practically made contact with the end of the cylinder (2) having the said tapered end (3) and wherein the said needle (4) has a guard (8) one of whose ends pivots on the base (4a) of the needle (4) the said guard being U-shaped and equal in length to or longer than the needle (4) ; the said guard (8) is sprung and moves between an unstable position when the syringe (1) is in use and where the said guard (8) is at an angle to the syringe and is held in place by the user, and a stable position where the said guard is in effect aligned with the needle (4) and covers the entire length of the same ; on the inside of the said guard (8) there is at least one, one-way locking element (9) running the length of the guard (8) flexible enough to permit the passage of the said needle (4) only when the said guard (8) springs back into its stable position ; the degree of flexibility of the said one-way locking element (9) is such as to prevent the introduction of the said needle (4) into the said guard (8) when the needle (4) is fitted with a protective cover (18) and is such as to prevent withdrawal of the uncovered needle (4) from the guard (8).

2. A disposable syringe as in claim 1 wherein, the said projection (7) has a wedge-shaped cross-section projecting towards the inside of the cylinder (2) and whose side surfaces (71, 7a) are at two different angles in relation to the generatrix of the cylinder (2) ; the surface (7a) of the projection (7) is closest to the end of the cylinder (2) with the tapered end (3) and has a steeper angle in order to permit plunger (5) movement beyond the said projection (7) when the plunger (5) is moving towards the needle (4) to expel the liquid contained in the cylinder (2).

3. A disposable syringe as in claim 1, wherein the said cylinder (2) has a single, continuous, ring-shaped projection (7) on the inner circumference of the said cylinder (2).

4. A disposable cylinder as in claim 1, wherein the said fixing means (10) consists of a mushroom head (11) coaxial to the said rod (6) and fixed to the same by the free end of the rod (12) and designed to fit into a seat (13) of a corresponding shape in the said plunger (5) ; the said mushroom head (11) has, on its side facing the said rod (6), a bevelled surface (15) which tapers towards the said rod (6) and which is designed to engage with and draw with it the walls of the said seat (13) in the plunger (5) as the latter is being moved away from the said needle (4) ; the opening angle of the said mushroom head (11) is such as to enable the plunger (5) to be drawn backwards during aspiration of the injection fluid into the cylinder (2) and is such as to prevent the said plunger (5), once it has passed the said projection (7) during its movement towards the needle (4), from travelling beyond the said projection (7) and to disengage the mushroom head (11) of the plunger (5) which remains trapped between the said projection (7) and the end of the cylinder (2) with the said tapered end (3).

5. A disposable syringe as in claim 1, wherein the said one-way locking element (9) consists of a strip (17) running lengthways along the inside of the said guard (8) at an angle to the arms of the guard (8) ; the said strip (17) is fixed to an arm of the said guard (8) along its outermost lengthways edge so that its free lengthways edge is close to the other arm of the said guard (8).

6. A disposable syringe as in claim 4, wherein the free lengthways edge of the said strip (17) is curved inwards towards the inside of the said guard (8) thus forming a hook (19) which traps the needle (4) inside the guard (8).

7. A disposable syringe as in claim 1, wherein the said guard (8) has two, one-way locking elements (9 and 16) which are independent from each other and which act independently on the needle (4) and are flexible in such a manner that they permit the entry of the needle (4) into the said guard (8) but prevent its subsequent withdrawal.

8. A disposable syringe as in claim 6, wherein the said one-way locking elements (9 and 16) each consist of a strip running lengthways along the inside of the said guard (18) at an angle to the said arms ; each of the said strips (17) is fixed to a respective arm of the said guard (8) on its outermost lengthways edge so that the lengthways edge closest to the other arm of the guard (8) is free ; the said strips (17) are at different angles with respect to the arms of the guard (8) and their innermost free lengthways edges are curved inwards towards the said guard (8) so as to form a hook (19) which traps the needle (4) inside the guard (8).

9. A disposable syringe as in claim 1, wherein the side of the said guard (8) facing the syringe (1) when the guard is in its stable position has a recess (20) which acts as a grip used to hold and keep the said guard (8) in its unstable position during use of the syringe (1).

EP 0 433 250 A2

**FIG 2**

17 4 8 20 4a 3 13 7 2 11 15 6 1 6

III III

A

8a

B

8 20

14

8

8 8a

C

**FIG 3**

9 17 19 17 16 4 19 8

**FIG 1**

18 4a 3 7a 7 7l 6 6

17

8

8a 20 2 7a 5 1